# EUROPEAN PATENT SPECIFICATION

(11) **EP 1 915 914 B1**
(45) Date of publication and mention of the grant of the patent: **08.08.2012**
(21) Application number: 07116356.2
(22) Date of filing: 13.09.2007
(51) Int. Cl.: A23L 1/304, A23G 9/32, A61K 33/06, A61P 19/10

(54) **Frozen confections**
Gefriersüssspeisen
Entremets glacés

(30) Priority: 03.10.2006 EP 06121668
(43) Date of publication of application: 30.04.2008
(73) Proprietor: Unilever PLC, London EC4Y 0DY (GB); Unilever N.V., 3013 AL Rotterdam (NL)
(72) Inventor: Berry, Mark, John, Bedford, Bedfordshire MK44 1LQ (GB); Hoddle, Andrew, Bedford, Bedfordshire MK44 1LQ (GB); Quail, Patricia, Jill, Bedford, Bedfordshire MK44 1LQ (GB); Wilkinson, Joy, Elizabeth, Bedford, Bedfordshire MK44 1LQ (GB)
(74) Representative: Warner, Guy Jonathan

(56) References cited:
- US-A1- 2003 147 995
- US-A1- 2004 040 448
- US-A1- 2006 067 986
- US-A1- 2006 141 103

## Description

### Technical Field of the invention

The present invention relates to frozen confections, in particular to frozen confections that are designed to promote bone health and are suitable for frequent consumption.

### Background to the invention

Bone health has been identified by the World Health Organisation as a major health issue (bulletin of the WHO, 2003, 81 p827). Approximately 75 million people in Europe, USA, and Japan suffer from bone diseases such as osteoporosis. This is in part because many people's diets do not contain sufficient calcium and other minerals (phosphorus, magnesium and zinc) and vitamin D. In particular, it is believed to be important to achieve adequate calcium intake during the years when bone is rapidly deposited (up to age 30). It has been has reported that the majority of teenagers (70-90%) do not consume enough milk (or other dairy products) to get sufficient calcium in their diet. This increases the risk of poor bone health in later life. Part of the reason is that dairy products such as milk and yoghurt are less appealing to many children and teenagers than other non-calcium containing products such as snacks and fizzy drinks.

Many foods, snacks and drinks have been fortified with micronutrients, including calcium and other minerals. Some of these products have been designed to be appealing to children and teenagers, for example ice creams. However, parents generally prefer their children not to eat ice creams every day because it contains high levels of saturated fat and added sugar.

US 6 030 650 discloses nutritional milk compositions such as milks and ice creams which are designed to include per serving size a specified percentage range of each essential micronutrient. US 2003 / 031758 discloses nutritional frozen desserts supplemented with micronutrients, such as vitamins and minerals. However, products that are fortified with a list of minerals can be unattractive to consumers as they may be perceived as "unnatural". Moreover, the addition of vitamins and minerals to ice cream can add unappetizing flavours, including bitter, metallic and sour. US 2003 / 0031758 overcomes this by adding an acidulant in order to reduce the off-flavour arising from the micronutrients. However this approach has the further drawback that the resulting products have an acidic, yoghurt-like taste. Moreover, although the supplemented products contain a significant proportion of the recommended daily amount of each of the micronutrients, this does not result in products that are optimized for bone health in terms of the balance of the micronutrients.
US 2006/141103 discloses frozen confections having a calorie level of 150 - 350 kcal/100g, which are nutritionally well balanced. These frozen confections do not contain minerals from a dairy source. Furthermore, the frozen confections according to US2006/141103 are nutritionally balanced and the sweetness of these confections does not appeal children who prefer sweet snacks.

Therefore, there remains a need for frozen confections which are optimized for bone health, attractive to consumers, especially children and teenagers and which are suitable for frequent consumption. Furthermore, the goal was to provide frozen confections with a low energy content but with a good sweetness level.

### Definitions

Definitions and descriptions of various terms and techniques used in frozen confectionery manufacture are found in Ice Cream, 6th Edition, Robert T. Marshall, H. Douglas Goff and Richard W. Hartel (2003), Kluwer Academic/Plenum Publishers. All percentages, unless otherwise stated, refer to the percentage by weight, with the exception of percentages cited in relation to the overrun.

### Frozen confection

As used herein, the term "frozen confection" is a confection intended for consumption in the frozen state (i.e. under conditions wherein the temperature of the confection is less than 0°C, and preferably under conditions wherein the confection comprises significant amounts of ice). Typical examples of frozen confections include ice creams, sorbets, water ices, fruit ices and milk ices.

### Sweetener

Sweetener means a sugar (i.e. mono and disaccharides), oligosaccharide containing from 3 to ten monosaccharide units joined in glycosidic linkage, glucose syrup with a dextrose equivalent (DE) of greater than 20, sugar alcohol, or a mixture thereof. Sweeteners include sucrose, fructose, lactose (for example from the source of milk protein), dextrose, invert sugar, corn syrup and sorbitol.

### Free sugars

As used herein, the term "free sugars" is defined as in "Diet, nutrition and the prevention of chronic diseases" - Report of a Joint WHO/FAO Expert Consultation, WHO Technical Report Series 916, WHO Geneva, 2003. Thus free sugars are all mono and disaccharides added by the manufacturer, cook or consumer plus sugar naturally present and sourced from honey, syrups and juices. Free sugars do not include sugars naturally present in and sourced from fruit or milk.

### Calculated estimate of sweetness

It is a well-known method to calculate the sweetness level. This can be done by summing the relative sweetness values of the individual sugar components multiplied by the proportion in which each component is in the product.
Tables of relative sweetness of sugars can be found in text book such as "Ice Cream Fourth Edition W.S. Arbuckle 1986" This reference is used for the calculation of the values in the examples.

### Dairy source of minerals

By a "dairy source of minerals" is meant a source of minerals produced from milk or milk products. Dairy sources include liquid milk, concentrated milk, milk powders, milk mineral compositions, whey, whey powders and/or whey protein concentrates/isolates, cream, butter, cheese, yoghurt and the like. The milk may come from cows or other mammals such as sheep, goats and buffaloes.

### Milk mineral composition

By "milk mineral composition" is meant a concentrated mixture of minerals (including calcium, phosphate, magnesium and zinc) sourced from milk or milk products. Milk mineral compositions may be prepared by the filtration of milk and milk derivatives, e.g. by the process described in European patent application EP 1 031 288 A1. Such compositions are available commercially. For example DMV International (Veghel, The Netherlands) supplies a milk mineral composition under the trade name Lactoval^{™}, Glanbia Nutritionals (Kilkenny, Ireland) under the name TruCal^{™} and ARLA Foods Ingredients (Skanderborgvej, Denmark) under the name CAPOLAC^{™}.

### Brief Description of the Invention

We have found that frozen confections which are designed to promote bone health may be formulated so that they are attractive, especially to children and teenagers, are suitable for everyday consumption, and do not suffer from the drawbacks of previous products, such as off flavour, by providing the essential micronutrients in the form of a dairy source.

Furthermore the frozen confections do have a good level of sweetness, at a low level of calories.

Accordingly, in a first aspect, the present invention provides a frozen confection having a calorie level of up to 150 kcal/100g comprising by weight of the frozen confection:
(i) at most 17% free sugar,
(ii) at most 5% total fat,
(iii) at most 3.5% saturated fat,
(iv) at least 0.5% protein,
(v) at least 0.3% calcium,
(vi) at least 0.15% phosphorus,
(vii) at least 0.015% magnesium,
(viii) at least 0.0005% zinc,
wherein the fat comprises butterfat,
characterized in that substantially all of the calcium, phosphorus, magnesium and zinc are from a dairy source. By "substantially all" it is meant that preferably at least 80% by weight of the calcium, phosphorus, magnesium and zinc are from a dairy source, more preferably at least 90%, most preferably at least 95%.

Dairy sources of minerals are considered natural and are therefore preferred by many consumers. Furthermore, frozen confections wherein substantially all of the calcium, phosphorus, magnesium and zinc are from a dairy source contain these minerals in essentially the same relative proportions as are found in milk. This is important because, for example, improper ratios of calcium to magnesium are believed to interfere with mineral absorption in the body.

Preferably at least 50% by weight of the calcium is provided in the form of a milk mineral composition.

A further finding of the present invention is that the use of a milk mineral preparation, wherein 90% or more of the milk mineral particles are smaller that 22µm prevents that the texture of the frozen confection gets "powdery" or "less smooth".
The use of the small particles allows that even at a high level of milk minerals, the texture of the frozen confection is good.
The size of the particles is defined by the largest dimension of the particle and can be determined according to well known methods.
Therefore a further embodiment of the present invention is a frozen confection wherein at least 90 % of the milk mineral particles have a size of less than 22µm.
The % is given in weight, based on the total weight of the milk mineral particles.

Preferably the frozen confection comprises from 3.3x10⁻⁶ to 3.3 x10⁻⁵% by weight vitamin D. More preferably the vitamin D is vitamin D₃.

Preferably the frozen confection according to the present invention has a maximum calorie level of 20 - 150 kcal/100g.

Preferably the amount of free sugars is less than 10% by weight of the frozen confection.

Preferably the frozen confection comprises lactose in an amount of at least 2% by weight of the frozen confection.

The frozen confection according to the present invention has a calculated sweetness of at least 13 %, preferably of at least 14 %.

Preferably the amount of total fat is from 0.5% to 4.0% by weight of the frozen confection.

Preferably the amount of long chain saturated fatty acids is less than 3% by weight of the frozen confection.

Preferably the amount of saturated fat is less than 3% by weight of the frozen confection.

Preferably the fat is butterfat.

Preferably the frozen confection does not comprise coatings or inclusions of chocolate, couverture, toffee, fudge or caramel.

Preferably the amount of calcium is at least 0.4%, the amount of phosphorus is at least 0.2%, the amount of magnesium is at least 0.02% and the amount of zinc is at least 0.0007% by weight of the frozen confection.
Preferably the ratio of the amounts of calcium to phosphorus is from 1:1 to 2.5:1.

In a related aspect, the present invention provides a frozen confection according to the first aspect of the invention in a portion of from 20 to 100g, and also a pack containing from 2 to 10 such portions.

### Detailed Description of the Invention

The frozen confections of the invention comprise water (some of which is present in the form of ice), sweeteners, fat and protein.

The amount of free sugars in the frozen confection is at most 17% by weight of the frozen confection, preferably less than 16%, more preferably less than 15%, even more preferably less than 14%, 12% or 10%, most preferably less than 8%. Foods suitable for everyday consumption should not contain high levels of free sugars.

However, in order to provide the customary sweetness associated with frozen confections and to avoid the confection being unduly hard, it is preferable that frozen confections comprise sweeteners in an amount of at least 10% by weight of the confection, more preferably at least 15%, most preferably at least 17%. To avoid the confection being too sweet, the amount of sweeteners should be at most 35%, preferably at most 30%, more preferably at most 25% by weight of the confection.

A preferred sweetener is lactose, because lactose aids calcium absorption and has a low relative sweetness. Lactose is particularly preferred when added as part of the milk solids because it is then not counted among the unhealthy free sugars. Thus it is preferable that lactose is present in an amount of at least 2% by weight of the frozen confection, preferably at least 3%, more preferably at least 5%. In order to avoid crystallisation of the lactose, however, it is also preferred that the lactose is present in an amount of less than 9%, preferably less than 8% by weight of the frozen confection.

In one embodiment of the invention, the frozen confection further comprises non-digestible saccharides, as such materials can contribute to the freezing point depression and/or body of the confection without increasing the sugar content of the confection or contributing to sweetness. Non-digestible saccharides are defined as those saccharides with a metabolisable energy content of less than 3 kcal (12.6 kJ) per g of saccharide. Common non-digestible saccharides are non-starch complex saccharides but others include resistant starches and non-digestible di-saccharides. Thus non-digestible saccharides include oligofructose, inulin, polydextrose, resistant starch and mixtures thereof.

Oligofructose and inulin are both available from the ORAFTI company under the trade names Raftlilose^{™} and Raftiline^{™}, respectively. Inulin and oligofructose are composed of linear chains of fructose units linked by β(2-1) bonds and often terminated by a glucose unit. Inulin contains chains with up to 60 fructose units. Oligofructose has between 2 and 7 fructose units. Oligofructose is obtained from inulin by partial enzymatic hydrolysis. Inulin has a metabolisable energy content (calorie conversion factor) of 1.2 kcal (5.0 kJ) g⁻¹, whilst oligfructose has a metabolisable energy content (calorie conversion factor) of 2 kcal (8.4 kJ) g⁻¹.Oligofructose is the preferred source of non-digestible saccharide for use in the present invention owing to its low molecular weight and therefore high freezing point depression power. It also believed that oligofructose and inulin may aid in calcium uptake.

Polydextrose is a randomly bonded condensation polymer of D-glucose with some bound sorbitol and citric acid. The 1,6-glycosidic linkage predominates in the polymer. Polydextrose is resistant to digestion in the human small intestinal tract and has a metabolisable energy content (calorie conversion factor) of 1.0 kcal (4.2 kJ) g⁻¹. It is available from the Danisco company under the trade name Litesse^{™}. Polydextrose has a relatively high molecular weight of around 2500.

Resistant starches are food starches or starch derivatives which are not digestible by the human body. There are four main groups of resistant starches: RS1, RS2, RS3 and RS4. RS1 is physically inaccessible starch, e.g. trapped in seeds. RS2 starch is granular starch. Examples include banana, high amylose starches. RS3 starch is a highly retrograded starch, e.g. extruded cereals. RS4 is chemically modified starch. Resistant starches have a metabolisable energy content (calorie conversion factor) of around 1.6 kcal (6.7 kJ) g⁻¹. Resistant starches are available commercially from National Starch under the trade names Novelose^{™} and Hi-maize^{™}.

Preferably the non-digestible saccharide is employed in an amount of at least 2% by weight of the frozen confection, preferably at least 3%, and most preferably at least 4%. In order to avoid undue freezing point depression and/or undue hardness it is preferred that the non-digestible saccharide is present in an amount of less than 15% by weight of the frozen confection, preferably less than 10% and most preferably less than 9%.

In order to provide the customary sweetness, the frozen confection may also comprise an intense sweetener, such as aspartame, saccharin, acesulfame K, alitame, thaumatin, cyclamate, glycyrrhizin, stevioside, neohesperidine, sucralose, monellin or neotame

Preferably the frozen confection comprises a small amount of total fat, such as at least 0.5% by weight of the frozen confection, preferably at least 1%. Frozen confections containing at least a small amount of fat are perceived as ice cream or milk ice type products and are more attractive to many consumers than completely fat-free frozen confections. However, the amount of total fat is at most 5% by weight of the frozen confection, preferably less than 4%, more preferably less than 3%, most preferably less than 2%. Foods suitable for everyday consumption should not contain high levels of total fat.

The frozen confection comprises at most 3.5% saturated fat. Preferably the amount of saturated fat is less than 3% by weight of the frozen confection, more preferably less than 2.5%, most preferably less than 2%. Foods suitable for everyday consumption should not contain high levels of saturated fat. In particular, the amount of long chain saturated fatty acids (i.e. fatty acids having acyl chain lengths of 16 carbon atoms or longer) is less than 3%, by weight of the frozen confection, more preferably less than 2.5%, most preferably less than 2%. It has been reported that long chain saturated fatty acids form insoluble calcium soaps under the alkaline conditions of the intestine, and therefore reduce the bioavailability of the calcium. The shorter the chain length, the more soluble the calcium soap.

Suitable sources for the fat include butterfat, palm oil, coconut oil, palm kernel oil, sunflower oil, safflower oil, linseed oil, soybean oil, walnut oil, corn oil, grape seed oil, sesame oil, wheat germ oil, cottonseed oil, olive oil, rapeseed oil (canola oil), peanut oil and mixtures thereof. Preferably the fat is butterfat. Butterfat contains a moderate amount of saturated fat (approximately 65%), and in particular contains significant amounts of short (C₄ to C₈) and medium (C₁₀ to C₁₄) chain saturated fatty acids.

The frozen confection comprises at least 0.5% protein. Preferably the amount of protein is at least 1% by weight of the frozen confection, more preferably at least 2% or 3%. Frozen confections containing at least these amounts of protein are more attractive to many consumers than completely protein-free frozen confections Suitable sources of protein (such as casein) include milk, concentrated milk, milk powders, and casein hydrolysates. These sources comprise casein micelles which contain calcium and phosphorus. Casein hydrolysates include casein phosphopeptides (CPPs). CPPs are available commercially, for example from DMV International under the trade name CE90CPP^{™}. Preferably the protein is casein or a casein hydrolysate. Many sources of casein are also good sources of calcium.

The frozen confections may also comprise one or more stabilisers. Suitable stabilisers include one or more of, gum arabic, gum ghatti, gum karaya, gum tragacanth, xanthan gum, guar gum, gelatine, agar, sodium carboxymethylcellulose, microcrystalline cellulose, methyl and methylethyl celluloses, hydroxypropyl and hydroxypropylmethyl celluloses, low and high methoxyl pectins, starches, maltodextrins, modified starches, and mixtures thereof. Preferably the frozen confections contain alginate, carrageenan, guar, locust bean gum or pectin or mixtures thereof in an amount of no more than 0.5% by weight of the frozen confection, more preferably no more than 0.3%, since these stabilisers are believed to interact with calcium, and may thereby reduce its bioavailability.The frozen confections may also comprise one or more emulsifiers. Suitable emulsifiers include mono- and di-glycerides of saturated or unsaturated fatty acids (e.g. monoglyceryl palmitate - MGP), polyoxyethylene derivatives of hexahydric alcohols (usually sorbitol), glycols, glycol esters, polyglycerol esters, sorbitan esters, stearoyl lactylate, acetic acid esters, lactic acid esters, citric acid esters, acetylated monoglyceride, diacetyl tartaric acid esters, polyoxyethylene sorbitan esters (such as polysorbate 80), sucrose esters, lecithin, egg yolk and mixtures of any these.

Preferably the frozen confection does not comprise coatings or inclusions of chocolate, couverture, toffee, fudge or caramel or the like since these typically contain high amounts of sugar and / or fat and therefore make the frozen confection less suitable for everyday consumption.

At least 50% of the calcium is provided in the form of a milk mineral composition, preferably at least 60% by weight of the calcium. In one embodiment, at least 70% or 80% is provided in the form of a milk mineral composition. Milk mineral compositions are suitable dairy sources and provide a convenient, concentrated source of calcium, as well as phosphorus, magnesium and zinc. We have found that this allows high proportions of the recommended daily amounts of these minerals to be provided in a low volume product. For example to obtain 1g of calcium (which is the RDA in many countries), one would have to consume about 800g of milk, whereas frozen confections of the present invention provide this amount of calcium in substantially smaller amounts, i.e. 330g or less.

Preferably the amount of calcium is at least 0.4% by weight of the frozen confection, more preferably at least 0.5%. In one embodiment, amounts of calcium of at least 0.6%, 0.7%, 0.8% or 0.9% are contemplated.

Preferably the amount of phosphorus is at least 0.2% by weight of the frozen confection, more preferably at least 0.25%, most preferably at least 0.3%. In one embodiment, amounts of phosphorus of at least 0.4% or 0.5% are contemplated.

Preferably the ratio of the amounts of calcium to phosphorus is from 1:1 to 2.5:1, more preferably from 1.5:1 to 2.5:1. Ratios of calcium to phosphorus in this range are found in milk and are believed to be most beneficial for bone health.

Preferably the amount of magnesium is at least 0.017% by weight of the frozen confection, more preferably at least 0.02%, most preferably at least 0.022%. In one embodiment, amounts of magnesium of at least 0.025% or 0.3% are contemplated.

Preferably the amount of zinc is at least 0.0007% by weight of the frozen confection, more preferably at least 0.001%, most preferably at least 0.0012%. In one embodiment, amounts of phosphorus of at least 0.0015% or 0.002% are contemplated.

It is expected that the greater the amount of calcium, phosphorus, magnesium and zinc in the frozen confection, the greater is the amount that will be taken up by the body.

Another important nutrient for bone health is vitamin D. Preferably the frozen confection comprises from 3.3x10⁻⁶ to 3.3 x10⁻⁵% vitamin D by weight of the frozen confection. More preferably the frozen confection comprises at least 3µg of vitamin D and/or derivatives thereof per 60g of the frozen confection (5x10⁻⁶% by weight of the frozen confection), most preferably at least 5µg (8.3x10⁻⁶%). In order to avoid unwanted physiological effects, the amount of vitamin D and/or derivatives thereof is more preferably less than 15µg per 60 g of the frozen confection (2.5x10⁻⁵%), most preferably less than 10µg (1.7x10⁻⁵%). (Quantities of vitamin D are sometimes also expressed in units of IU: 1 µg of vitamin D₃ is equivalent to 40 IU). Of the group of steroid molecules that are known as vitamin D, the preferred forms are vitamin D₃ (cholecalciferol) and/or derivatives thereof, vitamin D₂ (ergocalciferol) and/or derivatives thereof, and mixtures thereof. Particularly preferred is vitamin D₃ as it is the natural form of vitamin D.

Preferably the frozen confection does not contain oxalic acid which occurs in high levels in some plants such as rhubarb and spinach, or phytic acid, which can occur in high levels in plant seeds, such as cereals, legumes, and soy. Oxalic acid and phytic acid are generally considered to reduce the bioavailability of calcium.

The frozen confections of the present invention may be conveniently provided in single serve portions of from 20 to 100g, preferably from 25 to 75g. Portions of this size are suitable for consumption once per day and provide a significant proportion of the recommended daily amount of calcium, phosphorus, magnesium, zinc and optionally vitamin D. The present invention also provides a pack containing a plurality of such portions, for example from 2 to 10 portions. Packs containing a plurality of such portions are a convenient way of providing several days' supply of portions which are suitable for consumption once per day.

Owing to the relatively high levels of bio-available calcium provided by the frozen confections of the present invention, the frozen confections are capable of delivering enhanced amounts of calcium to the body, thereby assisting in providing the recognised benefits of an increased calcium intake. Thus the frozen confections of the present invention are particularly suitable for use to increase the calcium uptake in an individual; and/or to increase or maintain the density of bones and/or teeth in an individual; and/or to increase or maintain the strength of bones and/or teeth in an individual; and/or to improve or maintain the health of bones and/or teeth of an individual; and/or to assist the growth of bones and/or teeth in an individual; and/or to improve or maintain the appearance of teeth in an individual; and/or to treat osteoporosis; and/or to lower or maintain the blood pressure in an individual; and/or to assist in the control of the bodyweight of an individual; and/or to reduce the risk of colon cancer in an individual.

The frozen confections may also be used in the manufacture of a medicament for increasing the uptake of calcium in an individual; and/or increasing or maintaining the density of bones and/or teeth in an individual; and/or increasing or maintaining the strength of bones and/or teeth in an individual; and/or improving or maintaining the health of bones and/or teeth of an individual; and/or assisting the growth of bones and/or teeth in an individual; and/or improving or maintaining the appearance of teeth in an individual; and/or treating osteoporosis; and/or lowering or maintaining the blood pressure in an individual; and/or assisting in the control of the bodyweight of an individual; and/or preventing or reducing the risk of colon cancer in an individual.

In a further aspect, the present invention includes a method of increasing the calcium uptake by an individual, the method comprising administering to the individual a frozen confection according to the invention.

Frozen confections of the present invention may be prepared by any suitable process. However, in a further aspect of the invention there is provided a process for manufacturing the frozen confection, the process comprising the steps of:
(a) preparing a mix of ingredients according to the first aspect of the invention; then
(b) pasteurising and optionally homogenising the mix; then
(c) optionally ageing the mixture at between 0 and 5°C for at least 30 minutes; then
(d) freezing and optionally aerating the mix to produce the frozen confection.

Freezing may be under shear, for example in an ice cream freezer (scraped surface heat exchanger). In an ice cream freezer, aeration and freezing take place simultaneously. Ice creams are typically aerated to between 50 and 200% overrun, preferably to between 80 and 120% overrun. The partially frozen ice cream is extruded from the freezer at about - 5°C and then hardened e.g. in a hardening tunnel or blast freezer.

Alternatively freezing may be quiescent. By quiescent (or static) freezing, it is meant a process wherein the mix is cooled below its freezing point, such that partial or total solidification occurs through ice crystal formation, in the absence of an imposed shear field. Thus the liquid is frozen without it being deliberately agitated, mixed or shaken during freezing. Examples of quiescent processes include (but are not limited to) placing a container containing the mix into a cold environment and immersing a mould containing the mix in a bath of cold refrigerant. Unaerated / low overrun frozen confections, such as milk ice, are typically produced by quiescent freezing.

### Examples

The present invention will now be further described with reference to the following examples, which are illustrative only and non-limiting.

### Example 1: Ice cream and milk ices

Example 1 demonstrates ice cream and milk ice type frozen confections according to the invention. The formulation is given in Table 1 and its composition in terms of free sugar, fat, protein, minerals and vitamin D is shown in Table 2.

**Table 1: Formulation of example 1**

| **Ingredient** | **Weight (%)** |
|---|---|
| Skimmed milk powder | 11.9 |
| Sucrose | 15.2 |
| MD40 glucose syrup | 4.8 |
| Locust bean gum | 0.171 |
| Carrageenan L100 | 0.019 |
| Butterfat | 3.0 |
| HP60 emulsifier | 0.12 |
| Milk minerals | 0.93 |
| Vitamin D preparation | 0.005 |
| Vanillin | 0.014 |
| Water | to 100 |

The skimmed milk powder (SMP) was from Dairy Crest with a quoted typical composition of 52.0% lactose, 36.0% protein, 7.9% ash, 3.8% moisture and 1.25% maximum fat. It was measured to have mineral contents of: calcium 1.15%, phosphorus 0.96%, magnesium 0.11%, zinc 0.0041%. MD40 was 38DE spray-dried glucose syrup containing 5% water, 36.5% mono and disaccharides and 58.5% other solids, obtained from Cerestar (C*Dry GL 01934). Milk minerals were Capolac MM-0525 BG from Aria Foods, a natural concentrated fraction of minerals from milk containing a minimum of 24% calcium, ~11% phosphorus, ~0.6% magnesium, ~550ppm zinc. The vitamin D preparation was obtained from DSM as Dry Vitamin D3 100 CWS/AM and contained a quoted amount of 90 000 - 110 000 lU/g vitamin D3 (~2.5mg vitamin D /g of preparation).

**Table 2: Composition of example 1**

| **Component** | **Weight %** | **Amount per 60g portion** |
|---|---|---|
| Free sugar | 16.9 | 11.9g |
| Total fat | 3.0 | 1.8g |
| Saturated fat | 2.0 | 1.2g |
| Polyunsaturated fat | 0.09 | 0.05g |
| Protein | 4.3 | 2.6g |
| Casein | 3.8 | 2.3g |
| Calcium | 0.37 | 230 mg |
| Phosphorus | 0.22 | 130 mg |
| Calcium : phosphorus ratio | 1.7:1 | 1.7:1 |
| Magnesium | 0.020 | 13 mg |
| Zinc | 0.001 | 0.6 mg |
| Vitamin D | 1.3 x 10⁻⁵ | 7.5 µg |

Mixes with other flavours than vanilla were also prepared by using toffee flavour and colour (caramel), banana flavour and colour (turmeric), and strawberry flavour and colour (beetroot red).

The mixes were prepared as follows. Water at 75-80°C was added into a tank equipped with a turbo mixer. The dry ingredients, except the milk powder, were mixed together and added to the tank followed by the milk powder and then the fat, which had been pre-melted. The mix was blended for 5-10 minutes at 60-70°C. The mix was then homogenised at 140 bar and pasteurised at 82°C for 25 seconds in a plate heat exchanger. The mix was then cooled to 4°C in the plate heat exchanger and aged overnight in an ageing tank at 4°C, with gentle stirring.

Frozen confections were prepared by two routes.
(i) Ice cream-type products were prepared by freezing and aerating under shear. The mix was frozen using a scraped surface heat exchanger (Technohoy MF75) and was extruded at about -6°C with an overrun of 100%. On leaving the freezer, ice cream was collected in 500ml cardboard boxes or 150ml pots, blast frozen at -35°C for 3 hours and then stored at -25°C.
(ii) Milk ice-type products were prepared by quiescent freezing. The mix was poured into stainless steel moulds. Wooden sticks mounted in a holder were inserted. The moulds were placed in a glycol bath at -25°C until the mix was frozen. After freezing the moulds were immersed in warm water (25°C - 30°C) to release the frozen products from the moulds. The products were put in packets and stored in a freezer at -25°C.

All of the products tasted good. The aerated products were smooth and similar to typical low fat ice creams. The unaerated products were fairly soft in texture and similar to typical milk ice products.

### Example 2

Example 1 was repeated using higher amounts of milk minerals (2.9 and 3.5 wt% instead of 0.93 wt%) and without including the vitamin D preparation. The amounts of calcium, phosphorus, magnesium and zinc in are given in table 3.

**Table 3**

| **Amount (wt %)** | **2.9 wt% milk minerals** | **3.5 wt% milk minerals** |
|---|---|---|
| Calcium | 0.85 | 1.0 |
| Phosphorus | 0.48 | 0.56 |
| Calcium : phosphorus ratio | 1.8 | 1.8 |
| Magnesium | 0.039 | 0.039 |
| Zinc | 0.0024 | 0.0028 |

Products were prepared as described in example 1. On tasting, the products were found to be acceptable, and there was little difference between the sample with different amounts of milk minerals.

### Example 3

Example 1 was repeated using lower amount of sucrose and glucose syrup (14.0 and 4.6) instead of 15.2 and 4.8. The loss has been compensated by the addition of more water.

### Example 4: Ice Confection (Outside the scope of the invention)

Example 4 demonstrates an ice confection according to the invention. The formulation is given in Table 4 and its composition in Table 5.

**Table 4: Formulation of example 4**

| **Ingredient** | **Weight %** |
|---|---|
| Dextrose monohydrate | 3.80 |
| Sucrose | 13.30 |
| LBG | 0.20 |
| Citric acid | 0.45 |
| Orange flavour | 0.08 |
| Curcumin | 0.01 |
| Beta carotene | 0.015 |
| Hygel | 0.15 |
| Vitamin D preparation | 0.005 |
| Milk minerals | 2.52 |
| Sunflower oil | 0.60 |
| Sodium caseinate | 2.80 |
| Water | to 100 |

**Table 5: Composition of example 4**

| **Component** | **Weight %** | **Amount per 60g portion** |
|---|---|---|
| Free sugar | 16.8 | 10.1 g |
| Total fat | 0.6 | 0.36g |
| Saturated fat | 0.1 | 0.06g |
| Polyunsaturated fat | 0.4 | 0.24g |
| Protein | 2.51 | 1.5g |
| Casein | 2.51 | 1.5g |
| Calcium | 0.60 | 360mg |
| Phosphorus | 0.30 | 180 mg |
| Calcium : phosphorus ratio | 2.0:1 | 2.0:1 |
| Magnesium | 0.0151 | 9.1 mg |
| Zinc | 0.0014 | 0.8 mg |
| Vitamin D | 1.3 x 10⁻⁵ | 7.5 µg |

The ingredients were mixed in a stirred vessel, pasteurized (80°C for 15 secs) and homogenised, then cooled to +5°C and stored in a vessel in a +2°C room until used. Ice confections were prepared by three different routes
(i) quiescent freezing in metal moulds in a glycol bath at -25°C.
(ii) cooling the mix in a slush maker to -2°C, filling into cardboard tubes and blast freezing to -28°C.
(iii) freezing in a scrape surface heat exchanger (Technohoy MF75), before filling into cardboard tubes.

All of the products had an acceptable taste.

### Example 5: Very low sugar ice cream

Example 5 demonstrates a very low sugar ice cream according to the invention. The formulation is given in Table 6 and its composition in Table 7.

**Table 6: Formulation of example 5**

| **Ingredient** | **Weight %** |
|---|---|
| Sucrose | 5.00 |
| MD40 | 7.00 |
| SMP | 12.00 |
| Butterfat | 1.00 |
| Sunflower oil | 2.50 |
| LBG | 0.144 |
| Carageenan | 0.016 |
| Oligofructose | 7.00 |
| HP60 emulsifier | 0.12 |
| Aspartame | 0.01 |
| Milk Minerals | 0.93 |
| Vitamin D preparation | 0.005 |
| Water | to 100 |

Oligofructose was Raftilose P95 supplied by Orafti and had a moisture content of 3% (w/w). On a dry basis the Raftilose consisted of 95% (w/w) oligofructose and 5% (w/w) sugars (consisting of 3% sucrose, 1% fructose and 1% glucose).

**Table 7: Composition of example 5**

| **Component** | **Weight %** | **Amount per 60g portion** |
|---|---|---|
| Free sugar | 7.8 | 4.7 g |
| Total fat | 3.8 | 2.3 g |
| Saturated fat | 1.2 | 0.72 g |
| Polyunsaturated fat | 1.6 | 0.96 g |
| Protein | 4.3 | 2.6 g |
| Casein | 3.8 | 2.3 g |
| Calcium | 0.37 | 222 mg |
| Phosphorus | 0.23 | 138 mg |
| Calcium : phosphorus ratio | 1.6:1 | 1.6:1 |
| Magnesium | 0.02 | 12 mg |
| Zinc | 0.001 | 0.6 mg |
| Vitamin D | 1.3 x 10⁻⁵ | 7.5 µg |

Mixes with other flavours than vanilla were also prepared by substituting the vanillin in the mix with toffee flavour and colour (caramel), banana flavour and colour (beta carotene), and strawberry flavour and colour (beetroot red). The mixes were prepared as described in example 1, and then frozen using a Technohoy MF75 scraped surface heat exchanger, and was extruded at -4 to -5°C with an overrun of 100%. On leaving the freezer, ice cream was collected in 500ml cardboard boxes or 150ml pots, blast frozen at -35°C for 3 hours and then stored at -25°C. All samples tasted good and were found to be very acceptable for one-a-day consumption.
The various features and embodiments of the present invention, referred to in individual sections above apply, as appropriate, to other sections, *mutatis mutandis.* Consequently features specified in one section may be combined with features specified in other sections, as appropriate.

All publications mentioned in the above specification are herein incorporated by reference. Various modifications and variations of the described products, methods and uses of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are apparent to those skilled in the relevant fields are intended to be within the scope of the following claims.

### Sweetness test

As an important issue of the present invention is a low calorie level and a good sweetness of Example 3 is tested against a frozen confection from the prior art (Example 5 from US2006/014113) without milk minerals.
As described above the sweetness can be calculated according to the methods described in text books, but the sweetness of frozen confection is most accurately measured by sensory evaluation using a trained panel of human "tasters" or "assessors". Quantitative Descriptive Analysis (QDA^{®}) is a sensory descriptive technique, which was first developed by the Tragon Corporation (www.tragon.com) in the 1970's. In the examples below a trained panel consisting of 18 persons have scored the ice creams for sweetness on a scale of 1 (non sweet) to 10 (extremely sweet). The values in the table are the mathematical average of the values of the 18 persons. The composition of the comparison example 1 is listed in table 8:

**Table 8: Formulation of comparison example 1 (example 5 of US2006/014113)**

| **Ingredient** | **Weight %** |
|---|---|
| Skimmed milk powder | 8.1 |
| Palm oil | 1.89 |
| Rapeseed Oil | 1.02 |
| Glucose syrup (28 DE) | 24.1 |
| Emulsifier | 0.29 |
| Locust bean gum | 0.20 |
| Vanillin | 0.014 |
| Milk minerals | --- |
| Water | To 100 |

The following table show the result of the sweetness test:

**Table 9**

| **Example** | **Calories/100g** | **Sweetness (Panel)** | **Sweetness (calculated)** |
|---|---|---|---|
| Exp. 3 | 145 | 6.62 | 17 % |
| Comparison Exp 1 | 150 | 1.77 | 11 % |

The results clearly and surprisingly show that the frozen composition according to the present invention is far sweeter at a lower calorie level.

## Claims

1. A frozen confection having a calorie level of up to 150 kcal/100g comprising by weight:
(i) at most 17% free sugar,
(ii) at most 5% total fat,
(iii) at most 3.5% saturated fat,
(iv) at least 0.5% protein,
(v) at least 0.3% calcium,
(vi) at least 0.15% phosphorus,
(vii) at least 0.015% magnesium,
(viii) at least 0.0005% zinc,
wherein the fat comprises butterfat,
**characterized in that** substantially all of the calcium, phosphorus, magnesium and zinc are from a dairy source.

2. A frozen confection according to claim 1 wherein at least 50% by weight of the calcium is provided in the form of a milk mineral composition.

3. A frozen confection according to claim 2, wherein at least 90 % of the milk mineral particles have a size of less than 22µm.

4. A frozen confection according to claim 1,2 or claim 3 comprising from 3.3x10⁻⁶ to 3.3 x10⁻⁵% by weight vitamin D.

5. A frozen confection according to claim 4 wherein the vitamin D is vitamin D₃.

6. A frozen confection according to any of the preceding claim having a calorie level of 20 kcal/100g to 150 kcal/100g.

7. A frozen confection according to any one of the preceding claims wherein the amount of free sugars is less than 10% by weight of the frozen confection.

8. A frozen confection according to any one of the preceding claims wherein lactose is present in an amount of at least 2% by weight of the frozen confection.

9. A confection according to any one of the preceding claims having a calculated sweetness of at least 13 %, preferably of at least 14 %.

10. A frozen confection according to any one of the preceding claims wherein the amount of total fat is from 0.5% to 4.0% by weight of the frozen confection.

11. A frozen confection according to any one of the preceding claims wherein the amount of long chain saturated fatty acids is less than 3% by weight of the frozen confection.

12. A frozen confection according to any one of the preceding claims wherein the amount of saturated fat is less than 3% by weight of the frozen confection.

13. A frozen confection according to any one of the preceding claims wherein the fat comprises butterfat.

14. A frozen confection according to any one of the preceding claims which does not comprise coatings or inclusions of chocolate, couverture, toffee, fudge or caramel.

15. A frozen confection according to any one of the preceding claims wherein the amount of calcium is at least 0.4%, the amount of phosphorus is at least 0.2%, the amount of magnesium is at least 0.02% and the amount of zinc is at least 0.0007% by weight of the frozen confection.

16. A frozen confection according to any one of the preceding claims wherein the ratio of the amounts of calcium to phosphorus is from 1:1 to 2.5:1.

17. A frozen confection according to any one of the preceding claims in a portion of from 20 to 100g.

18. A pack containing from 2 to 10 portions of frozen confection according to claim 17.

## Patentansprüche

1. Gefrorene Süßware, die einen Kaloriengehalt von bis zu 150 kcal/100 g hat, umfassend nach Gewicht:
(i) höchstens 17 % freien Zucker,
(ii) höchstens 5 % Gesamtfett,
(iii) höchstens 3,5 % gesättigtes Fett,
(iv) wenigstens 0,5 % Protein,
(v) wenigstens 0,3 % Calcium,
(vi) wenigstens 0,15 % Phosphor,
(vii) wenigstens 0,015 % Magnesium,
(viii) wenigstens 0,0005 % Zink,
wobei das Fett Butterfett umfasst,
**dadurch gekennzeichnet, dass** im Wesentlichen alles von Calcium, Phosphor, Magnesium und Zink aus einer Milchquelle stammt.

2. Gefrorene Süßware gemäß Anspruch 1, wobei wenigstens 50 Gew.-% des Calciums in der Form einer Milchmineralstoffzusammensetzung bereitgestellt werden.

3. Gefrorene Süßware gemäß Anspruch 2, wobei wenigstens 90 % der Milchmineralstoffpartikel eine Größe von weniger als 22 µm haben.

4. Gefrorene Süßware gemäß Anspruch 1, 2 oder Anspruch 3, die 3,3x10⁻⁶ bis 3,3x10⁻⁵ Gew.-% Vitamin D umfasst.

5. Gefrorene Süßware gemäß Anspruch 4, wobei das Vitamin D Vitamin D₃ ist.

6. Gefrorene Süßware gemäß einem der vorangehenden Ansprüche, die einen Kaloriengehalt von 20 kcal/100 g bis 150 kcal/100 g hat.

7. Gefrorene Süßware gemäß einem der vorangehenden Ansprüche, wobei die Menge an freiem Zucker weniger als 10 Gew.-% der gefrorenen Süßware ist.

8. Gefrorene Süßware gemäß einem der vorangehenden Ansprüche, wobei Lactose in einer Menge von wenigstens 2 Gew.-% der gefrorenen Süßware vorliegt.

9. Süßware gemäß einem der vorangehenden Ansprüche, die eine errechnete Süße von wenigstens 13 %, vorzugsweise wenigstens 14 %, hat.

10. Gefrorene Süßware gemäß einem der vorangehenden Ansprüche, wobei die Menge an Gesamtfett 0,5 bis 4,0 Gew.-% der gefrorenen Süßware ist.

11. Gefrorene Süßware gemäß einem der vorangehenden Ansprüche, wobei die Menge an langkettigen gesättigten Fettsäuren weniger als 3 Gew.-% der gefrorenen Süßware ist.

12. Gefrorene Süßware gemäß einem der vorangehenden Ansprüche, wobei die Menge an gesättigtem Fett weniger als 3 Gew.-% der gefrorenen Süßware ist.

13. Gefrorene Süßware gemäß einem der vorangehenden Ansprüche, wobei das Fett Butterfett umfasst.

14. Gefrorene Süßware gemäß einem der vorangehenden Ansprüche, die keine Beschichtungen oder Einschlüsse aus Schokolade, Kuvertüre, Sahnekaramell, Fudge oder Karamell umfasst.

15. Gefrorene Süßware gemäß einem der vorangehenden Ansprüche, wobei die Calciummenge wenigstens 0,4 Gew.-% ist, die Phosphormenge wenigstens 0,2 Gew.-% ist, die Magnesiummenge wenigstens 0,02 Gew.-% ist und die Zinkmenge wenigstens 0,0007 Gew.-% der gefrorenen Süßware ist.

16. Gefrorene Süßware gemäß einem der vorangehenden Ansprüche, wobei das Verhältnis der Mengen an Calcium zu Phosphor 1:1 bis 2,5:1 ist.

17. Gefrorene Süßware gemäß einem der vorangehenden Ansprüche in einer Portion von 20 bis 100 g.

18. Packung, die 2 bis 10 Portionen gefrorener Süßware gemäß Anspruch 17 umfasst.

## Revendications

1. Dessert surgelé ayant une teneur en calories maximale de 150 kcal/100 g comprenant, en poids :
(i) au plus 17 % de sucre libre,
(ii) au plus 5 % de graisse totale,
(iii) au plus 3,5 % de graisse saturée,
(iv) au moins 0,5 % de protéine,
(v) au moins 0,3 % de calcium,
(vi) au moins 0,15 % de phosphore,
(vii) au moins 0,015 % de magnésium,
(viii) au moins 0,0005 % de zinc,
dans lequel la graisse comprend la matière grasse,
**caractérisé en ce que** pratiquement tout le calcium, le phosphore, le magnésium et le zinc provient d'une source laitière.

2. Dessert surgelé selon la revendication 1, dans lequel au moins 50 % en poids du calcium est fourni sous la forme d'une composition minérale de lait.

3. Dessert surgelé selon la revendication 2, dans lequel au moins 90 % des particules minérales de lait ont une taille inférieure à 22 µm.

4. Dessert surgelé selon les revendications 1, 2 ou la revendication 3 comprenant de 3,3x 10⁻⁶ à 3,3x10⁻⁵ % en poids de vitamine D.

5. Dessert surgelé selon la revendication 4, dans lequel la vitamine D est la vitamine D₃.

6. Dessert surgelé selon l'une quelconque des revendications précédentes ayant une teneur en calories de 20 à 150 kcal/100 g.

7. Dessert surgelé selon l'une quelconque des revendications précédentes, dans lequel la quantité de sucres libres est inférieure à 10 % en poids du dessert surgelé.

8. Dessert surgelé selon l'une quelconque des revendications précédentes, dans lequel le lactose est présent en une quantité d'au moins 2 % en poids du dessert surgelé.

9. Dessert selon l'une quelconque des revendications précédentes ayant un goût sucré calculé d'au moins 13 %, de préférence, d'au moins 14 %.

10. Dessert surgelé selon l'une quelconque des revendications précédentes, dans lequel la quantité de graisse totale est de 0,5 à 4,0 % en poids du dessert surgelé.

11. Dessert surgelé selon l'une quelconque des revendications précédentes, dans lequel la quantité d'acides gras saturés à longue chaîne est inférieure à 3 % en poids du dessert surgelé.

12. Dessert surgelé selon l'une quelconque des revendications précédentes, dans lequel la quantité de graisse saturée est inférieure à 3 % en poids du dessert surgelé.

13. Dessert surgelé selon l'une quelconque des revendications précédentes, dans lequel la graisse comprend la matière grasse.

14. Dessert surgelé selon l'une quelconque des revendications précédentes qui ne comprend pas d'enrobages ou d'inclusions de chocolat, de nappage, de toffee, de fudge ou de caramel.

15. Dessert surgelé selon l'une quelconque des revendications précédentes, dans lequel la quantité de calcium est d'au moins 0,4 %, la quantité de phosphore est d'au moins 0,2 %, la quantité de magnésium est d'au moins 0,02 % et la quantité de zinc est d'au moins 0,0007 % en poids du dessert surgelé.

16. Dessert surgelé selon l'une quelconque des revendications précédentes, dans lequel le rapport des quantités de calcium au phosphate est de 1:1 à 2,5:1.

17. Dessert surgelé selon l'une quelconque des revendications précédentes sous la forme d'une portion de 20 à 100 g.

18. Boîte contenant de 2 à 10 portions de dessert surgelé selon la revendication 17.
